# EUROPEAN PATENT APPLICATION

(11) **EP 4 681 642 A1**
(43) Date of publication of application: **21.01.2026**
(21) Application number: 24189758.6
(22) Date of filing: 19.07.2024
(51) Int. Cl.: A61B 5/16, A61B 5/00

(54) **PORTABLE DEVICE FOR REAL-TIME EXPERIENCE LOGGING AND INTERVENTION, SYSTEM COMPRISING A PORTABLE DEVICE AND A WEARABLE, AND USE IN ECOLOGICAL MOMENTARY ASSESSMENT**

(71) Applicant: medigital GmbH, 58638 Iserlohn (DE)
(72) Inventor: Van Aken, Nina, 58638 Iserlohn (DE); Dunbar, Allan, 58638 Iserlohn (DE); Kotowski, Nick, 58638 Iserlohn (DE); Korsak, Alexandr, 58638 Iserlohn (DE)
(74) Representative: Hamm&Wittkopp Patentanwälte PartmbB

(57) **Abstract**

The present invention relates to a portable device which is configured to capture manual input from a user based on the user's real time or near real time experience with a memory, and at least two tactile inputs. The data captured by the portable device may be processed by an evaluation unit and utilised in Ecological Momentary Assessment (EMA). The portable device of the present invention allows experience logging in a practical, convenient, and discrete way. Further covered by the invention is a system comprising a portable device and a wearable and the use of a portable device or a system for capturing a user's experiences for EMA.

## Description

The present invention relates to a portable device which is configured to capture manual input from a user based on the user's real time or near real time experience. The portable device comprises a memory, and at least two tactile inputs. The memory is configured to capture at least the number of manual inputs. The data captured by the portable device may be processed by an evaluation unit and utilised in Ecological Momentary Assessment. The portable device of the present invention allows experience logging in a practical, convenient, and discrete way. Furthermore, the present invention includes a system comprising a portable device and a wearable. The portable device may utilise vibrational cues triggered by various events, such as physiological data from the wearable. Moreover, the present invention relates to the use of a portable device or a system for capturing a user's experiences for Ecological Momentary Assessment.

### Technical background and prior art

Ecological Momentary Assessment (EMA) is a research method designed to capture individuals' psychological states, behaviours, and environmental contexts as they naturally occur. By collecting data in real time or near real time, EMA offers insights into the day-to-day experiences of participants within their usual environments.

EMA addresses several limitations of traditional psychological and behavioural research methods, which often rely on participants recalling past experiences. Such approaches can be prone to recall bias and lack ecological validity, as they might not accurately reflect individuals' actual environments or experiences.

EMA has several benefits vis-à-vis traditional methods. For example, by collecting data as events happen or shortly thereafter, EMA minimises the inaccuracies caused by participants forgetting details or their memories being influenced by recent events or moods. The data gathered in natural settings ensures that research findings are more applicable to real-world experiences. The frequent data collection allows for the observation of psychological and behavioural changes over time, providing a dynamic picture of human experiences. Questions about participants' environments and interactions offers deeper insights into what affects psychological states and behaviours; thereby moments of stress or need in real time supports the delivery of interventions when they are most needed can be identified, enhancing the effectiveness. This allows a detailed, individual-level data over time which aids in creating tailored treatments and interventions. Additionally, this approach can enable just-in-time interventions, providing immediate support based on real-time data, thereby increasing the likelihood of positive outcomes. Performing the EMA itself can act as a just-in-time intervention by prompting participants to reflect on their current state and behaviours, potentially mitigating negative experiences or reinforcing positive ones.

However, EMA also has several shortcomings. For example, interacting with devices can disrupt daily activities of the participants, e.g. interacting with a touch screen. Charging needs and technical issues can interrupt data collection. The volume and complexity of data require sophisticated analysis, challenging interpretation. Accurately pinpointing intervention moments requires advanced data interpretation, with risks of timing errors. Misjudging the context can render interventions inappropriate or ineffective. Balancing the frequency of interventions to avoid overwhelming participants is difficult. Interventions may be perceived as intrusive if they disrupt or are poorly timed, potentially reducing engagement.

Currently, a variety of tools and technologies are employed for EMA, including
- smartphone applications which prompt users for input at specific times or in response to certain events, utilising features like notifications for timely data collection,
- wearable devices like smartwatches which gather physiological data and can prompt for psychological or environmental context inputs,
- SMS and Interactive Voice Response (IVR), that use text messages or phone calls to collect data,
- specialised electronic diaries and web-based online surveys that facilitate data entry at various times,
- photographs, videos, audio recordings, and passive sensor data from devices which offers rich contextual information and minimizes active participant effort.

Current methods have been mainly designed for research purposes and present several issues especially when considering wide spread use. For example, smartphone and web-based EMA applications require frequent and time-consuming surveys which are often inconvenient and lead to decreased participation of users resulting in poor data quality. Furthermore, responding in real-time is not always feasible in daily life. Wearable devices such as smartwatches are often limited in capturing complex emotions and thoughts without supplemental methods and the continuous wear can be uncomfortable, affecting participant compliance negatively. Depending on the device type and usage conditions physiological data accuracy can vary.

US2023104450A1 discloses a system for determining a mental health state of a user. US11456080B1 discloses a method for data collection for disease management. EP4163927A1 discloses a system for determining a user's mental health state using a smart phone. Actuating a smart phone, however, requires the user to visually engage with the smart phone when inputting data which is sometimes not desired because users often seek for a discrete solution for capturing theirs experience.

The current focus on research-driven EMA highlights the need for simpler, more practical, and discrete solutions for wider applications.

### Object of the invention

The object of the present invention was the provision of an opportunity to allow users a quick and discrete capture of experiences such as emotional states, cognitive processes, physical sensations, social interactions, spiritual experiences, and behaviour. The users shall be enabled to capture their experiences in a discrete, user-friendly, and accurately manner without any difficulties. Similarly, interventions should be timely, minimal, and effectively tailored to individual needs, striking a balance between utility and user experience.

These and other shortcomings are solved by the subject matter of the independent claims. Preferred embodiments are part of the dependent claims or further explained below.

### Description of the invention

Traditional methods for assessing emotional and cognitive states often fall short due to their time-consuming nature, lack of convenience, and insufficient discretion for effective EMA. To address these challenges, a novel portable device designed for both portability and discretion is proposed, making it perfect for discreet use in any setting.

The present invention concerns a portable device designed for the convenient and discreet monitoring and logging of human experiences, including emotional states, cognitive processes, physical sensations, social interactions, spiritual experiences, and behaviour, based on a participant's input. The device according to the invention is a portable device that is configured to capture data from manual inputs from a user. It comprises at least a memory and two tactile inputs; the memory is configured to capture the data in form of at least the number of manual inputs. This portable device allows for in-situ recording of emotions and cognitive events, providing timely interventions to enhance emotional regulation.

Human experience in the sense of the present invention covers:
- emotions which involve complex reactions such as happiness and sadness,
- cognitive processes including mental actions like memory and decision-making,
- physical sensations such as hunger or pain,
- social interactions such as empathy and conflict,
- spiritual experiences which may involve feelings of connectedness or reflections on life's purpose, and
- behaviour including adaptive, maladaptive, and social actions, learning and habitual routines, compulsive actions, and various forms of communication.

The device according to the invention is suitable for use in a variety of environments, including public spaces. Users can log events, preferably along with their precise time and date, by making a manual input. The manual input is captured in a memory and may be transferred to an evaluation unit or computing platform, like a smartphone, laptop, smartwatch, tablet computer etc. for analysis or further intervention. The memory is capable of capturing data from at least the number of manual inputs for each of the at least two tactile inputs separately. The inventive portable device is compact, and small enough to be easily carried in a pocket or worn discreetly and can be conveniently carried.

In a preferred embodiment, the tactile input is a button. In a further preferred embodiment, the portable device comprises at least two or more buttons. The buttons may be uniquely shaped, textured, sized, or positioned, to allow users to log events through touch alone, without the need to visually engage with the device. The uniquely shape of the buttons enables the user to make manual input even with the device being in a pocket. In this preferred embodiment, the user can distinguish the two or more buttons of the portable device by their shape, texture, position, or size.

In one embodiment, the memory of the portable device is a mechanical storage. In a preferred embodiment, the portable device is an electronic portable device and the memory is preferably an electronic memory. The memory is configured to capture at least the number of manual inputs for each of the at least two tactile inputs separately. In a preferred embodiment it is possible to engage the portable device with an evaluation unit, such as a computer or a smartphone, a smartwatch, or a tablet computer via taking a picture of the device and performing image analysis on the evaluation unit. In another embodiment, the data captured on the memory of the portable device can be read by a camera from a smart phone or a tablet computer and processed by an app or computer program.

The device according to the invention can be customised for various monitoring requirements, with functionalities tailored for specific therapeutic focuses, through different device designs or customizable buttons. In a preferred embodiment the device includes internal stimuli, such as vibrations or sounds, to prompt user interaction or guide them in assessing their emotional or cognitive state. This feature expands the device's applications, including emotional state tracking, cognitive process monitoring, and support for various health and wellness applications through real-time data collection and analysis. In one embodiment, a stimulus is generated by a vibration motor which is configured to express a stimulus in the form of vibrations. In another embodiment, a stimulus is generated by a signal generator which is configured to express a stimulus in the form of one or more beeps. In another preferred embodiment, the stimulus is expressed in response to a manual input, most preferably in the form of a vibration.

The device features tactile inputs, such as uniquely shaped, textured, sized, or positioned buttons, allowing users to easily identify and log emotional or cognitive events through touch alone. This discreet logging capability, without the need to visually check the device, renders it ideal for a variety of environments, including public spaces.

When a user experiences a notable emotional state or wishes to evaluate a cognitive process, pressing the appropriate button logs the event, preferably along with its precise time and date on the memory. This data can then be transferred to a computing platform, like a smartphone, via for example, wireless connection or Bluetooth when available, where it can be analysed or used for further intervention through applications designed for digital therapeutics or monitoring, based on the user's specific needs.

Furthermore, the data collected from this device could be combined with other data measured by a smartphone for example, GPS location, or physiological data from a wearable. This combined information would enhance understanding of emotional or cognitive events.

Preferably the portable device is an electronic portable device. In this configuration, the memory is preferably an electronic memory, more preferably an electrical data storage. The memory is configured to be synchronised with an evaluation unit. Preferably, the portable device is equipped with sufficient memory to store data between sync intervals, and can log enough experiences to ensure no data loss during periods without connectivity.

It can be desired that the electronic portable device further comprises an interface which is preferably configured to enable communication between the memory and an evaluation unit, for example via an USB port, or more preferably via wireless communication (WiFi) or Bluetooth. Preferably the evaluation unit is a computing platform such as a PC, a smartphone, or a tablet computer. The portable device may comprise either a rechargeable battery or a non-rechargeable battery which can last for approximately one year or longer.

In a further preferred embodiment, the electronic portable device is configured to capture the time and/or the date of the manual input on the memory. Users can later analyse this data via an evaluation unit to identify triggers, evaluate coping strategies, and track their progress over time, enhancing their emotional well-being through personalised feedback and a simplified approach to understanding emotional patterns. The memory is capable to capture at least the number of manual inputs for each of the at least two tactile inputs separately.

In a preferred embodiment, the portable device comprises at least three buttons. In this embodiment, using to decide which button to press based on one's emotional state acts as an immediate emotional regulation tool. In this exemplary configuration, the device according to the invention has one button for positive energy that captures moments of happiness or contentment, one button for negative energy that records times of sadness, anger, or frustration, and one button for strong emotions that logs overwhelming emotions. Such a setup enhances the device's ability to capture a wide range of emotional experiences.

This decision-making process introduces mindfulness and self-reflection. Pausing to think about which button to press helps users detach from their immediate emotions. Further, identifying the nature of emotions increases awareness and understanding of emotional patterns. Choosing a button can shift the user from emotional to rational thinking, aiding in emotional management. The collected data reveals trends and triggers, aiding in the development of coping strategies. Insights from the data help tailor more effective, personalized interventions for emotional well-being.

For participants with memory challenges, maintaining a consistent daily routine can be daunting. A preferred version of the portable device according to the invention aids in this aspect. Such a preferred portable device for example can have five buttons, each symbolizing essential daily tasks such as medication intake, meals, hydration, physical activity, and bedtime. The distinctive textures and/or shapes of these buttons support easy tactile recognition. By pressing the corresponding button post-activity, participants reinforce structured routine habits. This logged information, once uploaded to an app, allows caregivers to monitor the individual's daily adherence and well-being.

In another preferred embodiment, the portable device may assist users with ADHD in better managing time and tasks by promoting self-monitoring and enabling data analysis for future adjustments. With five buttons designated to various challenging categories such as work/study tasks, daily routines, breaks, leisure activities, and moments of distraction, users can log activities throughout the day based on tactile recognition.

Reviewing this collected data alone or with a therapist or coach uncovers behavioural patterns, pinpointing productive periods and highlighting potential for strategy refinement. This analysis forms the basis for actionable changes, like reallocating tasks to more focused times or setting specific, achievable goals aimed at enhancing productivity and focus.

The portable device according to the invention can be customised to a broad spectrum of emotional and cognitive monitoring requirements and include simple yet effective timely interventions. Empowering users with such logging control and timely interventions, the proposed device not only respects privacy but also encourages accurate self-reporting, making it an invaluable tool for personal development and emotional and cognitive health management.

In a further preferred embodiment, the device has stimuli through vibration or sounds it makes. For discretion vibration is preferred. The device as a whole could vibrate as a prompt for the user to perform an action with the buttons. Alternatively, each button could vibrate individually. It can be preferred that stimuli can be turned on/off manually via a switch or by an external evaluation unit like a computer or smart phone or tablet computer connected to the portable device. It is also possible to customize stimuli patterns directly through the evaluation unit. This includes creating and assigning specific patterns to different types of notifications or emotional/cognitive event logs.

In one embodiment, the device vibrates at random or scheduled times throughout the day as a prompt for the user to assess and log their current emotional state using the buttons. This encourages regular reflection on emotional states, fostering greater emotional awareness.

In another embodiment, during an emotional event, individual button vibrations can signal the user to identify and log the intensity and nature of their emotions (e.g., happiness, sadness, anxiety). This immediate logging helps capture the emotional state more accurately than retrospective reporting.

The device's vibrational cues could play a role in fostering mindful decision-making and emotional processing. By prompting users to actively engage with the device during moments of emotional significance, it encourages a mindful pause, aiding in the reduction of immediate emotional intensity. Additionally, the capability for quick, subsequent check-ins allows for immediate follow-up support. This could include reminders for breathing exercises, prompts to engage in physical activity, or cues to practice grounding techniques, offering timely interventions tailored to the user's emotional needs.

The benefits are particularly important for in monitoring cognitive processes. Monitoring the user's ability to respond to vibration prompts can assess sustained attention and focus levels, especially if the vibrations are subtle or occur amidst distractions. By using patterns of vibrations that the user must remember and replicate through button presses, the device can evaluate working memory capacity. Tasks that require the user to follow multi-step instructions based on specific vibration patterns can assess executive functions, including planning, problem-solving, and cognitive flexibility. The user's ability to distinguish between different vibration patterns tests sensory processing skills, which involve interpreting sensory information efficiently. Measuring the time it takes for a user to respond to a vibration prompt can assess their reaction time, providing insights into cognitive speed and motor coordination. If the device uses spatially distributed vibrations (e.g., individual buttons vibrating in a specific order), it can be used to evaluate the user's spatial memory and their ability to navigate and remember spatial relationships. The ability to process sequences of vibrations and replicate them through button presses tests sequential processing capabilities, which may be important for understanding temporal sequences and patterns.

By requiring the user to make quick decisions based on the type of vibration felt (e.g., pressing a different button depending on the vibration pattern), the device can assess decision-making speed and accuracy. Tracking improvements in performance over time on tasks involving vibration cues can provide insights into the user's learning curve and memory consolidation processes. Assessing the user's ability to respond to different sounds or auditory sequences could measure auditory processing capabilities and auditory memory.

The portable device according to the invention allows for fine-tuning the understanding of the impact of ADHD medications on users throughout the day. By tracking attention levels at various times, users and healthcare providers can observe how medication effectiveness fluctuates, aiding in the optimization of daily schedules and medication regimens. For example, it is possible to identify peak performance times to align demanding tasks like work or study, ensuring activities are planned when the user is most responsive. Further, with the aid of the device according to the invention therapists can obtain insights into optimal medication timing and dosing to maintain consistent attention levels, enhancing overall effectiveness and reducing potential side effects.

Furthermore, the present invention relates to a system comprising the inventive portable device and a wearable. Preferably, the wearable is any of a smart phone, a smart watch, or a fitness tracker. In this embodiment, the portable device and the wearable may be connected via an USB-port or wireless, for example via Bluetooth.

In a further preferred embodiment, the system includes an evaluation unit which may process the data captured on the memory of the inventive portable device. The evaluation unit may be any of a computing platform such as a PC, a smartphone, or a tablet computer. The evaluation unit may be configured to process the data captured on the memory of the inventive portable device, for example using an appropriate programme installed on the evaluation unit. In one embodiment, the wearable and the evaluation unit are one device, i.e., the wearable is also an evaluation unit.

In another preferred embodiment, the wearable is capable of processing the data captured on the memory of the inventive portable device. More preferably, the wearable is configured to process the data on an app. For example, the app may visualise the data in an appropriate form helping the user to interpret the captured data.

In one embodiment, the wearable is configured to trigger a stimulus on the inventive portable device. In a preferred embodiment, the wearable comprises one or more sensors which are configured to detect a user's physiological parameters. These include *inter alia:*
- the heart rate (bpm), measured by a heart rate sensor of the wearable; the heart rate variability derived indicates autonomic nervous system activity and stress levels;
- the blood oxygen level (SpO2), measured by a SpO2 Sensor (Pulse Oximeter); based on fluctuations in blood oxygen levels during sleep apnoea can be detected;
- electrical activity of the heart, measured by an ECG sensor; allows the detection of atrial fibrillation (AFib) and other cardiac arrhythmias;
- acceleration forces in three dimensions, measured by an accelerometer; allows to monitor for example the step count, physical activity level, and fidgeting, which can indicate nervousness or restlessness;
- angular velocity in three dimensions, measured by a gyroscope; allows gait analysis and balance assessment, indicating stability and coordination;
- geolocation data, measured by a GPS-sensor; allows to measure the travelled distance and speed, which might be used to assess endurance and performance in outdoor activities;
- atmospheric pressure, measured by a barometer; allows to determine elevation change, used to measure floors climbed and altitude, relevant for outdoor and fitness activities;
- skin temperature, measured by a temperature sensor; allows fever detection, which can indicate an illness or infection;
- sound levels and voice input, measured by a microphone; allows to detect snoring, which can be used to monitor sleep quality and detect sleep apnoea; light exposure, measured by a ambient light sensor; allows circadian rhythm analysis, which can be used to optimize sleep patterns and overall well-being;
- blood flow using photoplethysmography (PPG), measured by an optical sensor; allows to identify stress levels, derived from analyzing blood flow patterns and variability;
- skin conductance, measured by an Electrodermal Activity (EDA) Sensor; allows to detect stress, based on changes in skin conductance related to sweat gland activity.

In one embodiment, the wearable is configured to detect a stress level of the user. The stress level is preferably determined by the heart rate or the skin conductivity. Based on the determined physiological parameter, the wearable may be preferably configured to trigger a stimulus on the portable device according to the invention.

In a preferred embodiment, the wearable or the evaluation unit may comprise a GPS sensor. In this embodiment, the wearable or the evaluation unit may be configured to communicate the GPS location to the portable device or the memory of the portable device. The GPS location may be included into the data. In another embodiment, the GPS location is stored on the wearable or the evaluation unit and processed by the wearable or by the evaluation unit in combination with the data from the memory of the portable device.

Moreover, the present invention relates to a use of the inventive portable device or the inventive system in EMA by capturing a user's experience in the form of emotional states, cognitive processes, physical sensations, social interactions, spiritual experiences, or behaviour. For example, the portable device and/or the system may assist ADHD individuals in strategising daily activities based on real-time attention span data. The processed data may provide attention level insights which are helpful to plan daily activities, ensuring tasks requiring higher concentration.

The invention is further exemplified by means of the following figures which disclose:
**Fig. 1****:** a scheme of portable devices having different tactile inputs,
**Fig. 2****:** a scheme of a portable device having three and five different tactile inputs,
**Fig. 3****:** a scheme of a portable device having three tactile inputs,
**Fig. 4****:** a scheme of portable devices including stimuli patterns,
**Fig. 5** and **Fig. 6****:** a scheme of a system.

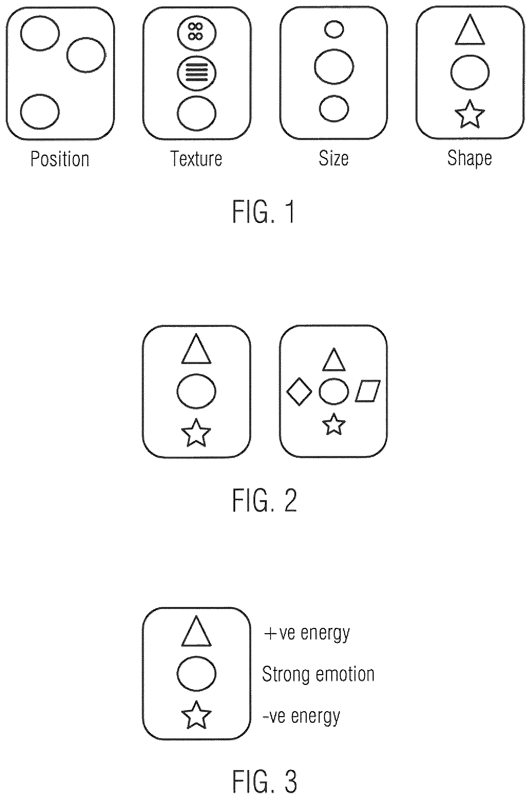

**Fig. 1** discloses in total four schemes of portable devices according to the invention. The portable devices have three tactile inputs each, in the form of buttons. The buttons differ in their position, texture, size, and shape, respectively. The configuration allows a user-friendly actuation of the buttons without visual engagement.

**Fig. 2** shows two schemes of portable devices according to the invention. The portable devices have three and five tactile inputs in the form of buttons, respectively. The buttons differ in their position and shape and allow a user-friendly actuation of the buttons without visual engagement.

**Fig. 3** discloses a scheme of portable device according to the invention. The portable device has three tactile inputs in the form of buttons. The buttons differ in their position and shape. In this exemplary scheme, the upper button logs overwhelming emotions, the middle button captures moments of happiness or contentment, and the lower button records times of sadness, anger, or frustration. The memory (not displayed) is capable to capture the number of activations of each button including the date and time.

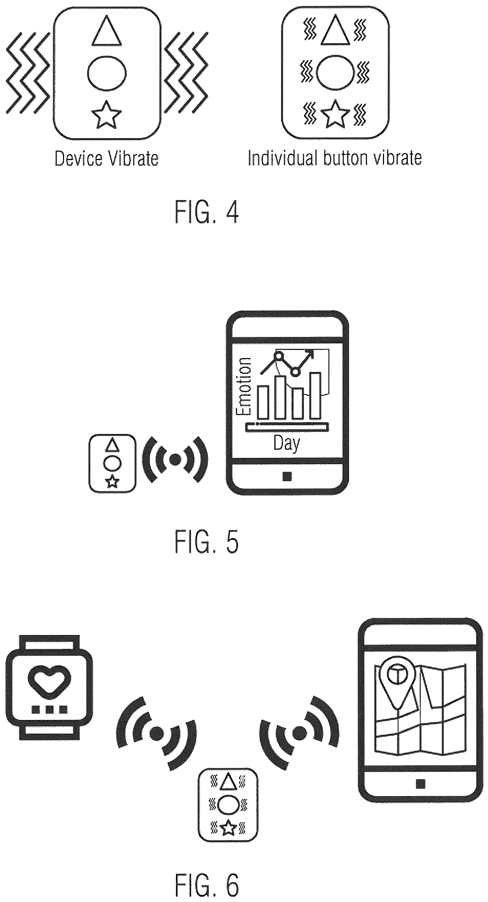

**Fig. 4** discloses two schemes of portable devices including internal stimuli, for example in the form of a vibration or in the form of a beep. In this configuration, the portable device may include a vibrational motor or a signal generator which are capable to prompt a stimulus. The portable device self might vibrate or give a beep; alternatively, the vibration or beep is triggered by a user's manual input of the buttons.

**Fig. 5** shows a system according to the invention comprising a portable device and a wearable, wherein the wearable is a smartphone. The portable device and the wearable are connected via Bluetooth and the data captured by the memory of the portable device is processed on the wearable.

**Fig. 6** shows another system according to the invention including a portable device, a wearable, and an evaluation unit. The wearable is a smartwatch and the evaluation is a smartphone. The smartwatch and the smartphone are connected with the portable device via Bluetooth. The smartwatch monitors the heartrate of the user and triggers a stimulus on the portable device based on said heart rate. The smartphone renders the position of the user via GPS.

## Claims

1. A portable device configured to capture data from a manual input from a user, the portable device comprising at least:
- a memory, and
- two inputs,
wherein the memory is configured to capture data in form of at least the number of manual inputs, and wherein the at least two inputs are tactile inputs.

2. The portable device according to claim 1, wherein the two or more tactile inputs are in the form of a button, wherein preferably the two or more tactile inputs differ in their shape and/or texture and/or size.

3. The portable device according to claim 1 or 2, wherein the portable device is an electronic portable device, wherein preferably the memory is an electronic memory, more preferably an electrical data storage.

4. The portable device according to claim 3, further comprising an interface, wherein preferably the interface is configured to enable communication between the memory and an evaluation unit, preferably via WiFi or Bluetooth.

5. The portable device according to anyone of the preceding claims, wherein the memory is configured to capture the time and/or the date of the manual input.

6. The portable device according to anyone of the preceding claims, wherein the portable device comprises
- a vibrational motor that is configured to express a stimulus in form of a vibration, preferably in response to a manual input, or
- a signal generator that is configured to express a stimulus in form of a beep, preferably in response to a manual input, or
- both,
wherein preferably the stimulus is a vibrational signal.

7. The portable device according to claim 6, wherein the tactile input expresses a stimulus upon a manual input, wherein preferably the stimulus is a vibrational signal.

8. A system comprising a portable device according to anyone of claims 1-7 and a wearable, wherein the wearable preferably is a smartphone or a smartwatch or a fitness tracker.

9. The system of claim 8, further comprising an evaluation unit, wherein the evaluation unit is capable to process the data from the memory.

10. The system of claim 9, wherein the evaluation unit is a computer or a smartphone or a smartwatch or a tablet computer.

11. The system of anyone of claims 8 to 10, wherein the wearable is capable to process the data from the memory.

12. The system of anyone of claims 8 to 11, wherein the wearable comprises one or more sensors, wherein the one or more sensor is configured to detect physiological parameters, wherein preferably the physiological parameters are selected from one or more of the group consisting of: heart rate (bpm), blood oxygen level (SpO2), electrical activity of the heart, acceleration forces, angular velocity, geolocation data, atmospheric pressure, skin temperature, sound levels, light exposure, blood flow, and skin conductance.

13. The system of anyone of claims 8 to 12, wherein the wearable or the evaluation unit is configured to trigger a stimulus on the portable device, preferably depending on the physiological parameters measured by the wearable, or the analysis of the data captured on the memory of the portable device.

14. The system of anyone of claims 8 to 13, wherein the wearable or the evaluation unit comprise a GPS-sensor, wherein the wearable or the evaluation unit are configured to communicate the data from the GPS-sensor to the portable device, wherein preferably the data from the GPS-sensor is recorded with a time stamp on the evaluation unit or the wearable, and wherein more preferably the GPS data is combined with data from the portable device.

15. Use of a portable device according to anyone of claims 1-7 or a system according to anyone of claims 8 to 14 in Ecological Momentary Assessment (EMA) by capturing a user's experience in the form of emotional states, cognitive processes, physical sensations, social interactions, spiritual experiences, or behaviour.
